# EUROPEAN PATENT APPLICATION

(11) **EP 1 004 294 A1**
(43) Date of publication of application: **31.05.2000**
(21) Application number: 99203783.8
(22) Date of filing: 12.11.1999
(51) Int. Cl.: A61K 9/02, A61K 9/06, A61K 47/32, A61K 31/21

(54) **Pharmaceutical composition comprising a nitric oxide (NO) donating organic compound**

(30) Priority: 27.11.1998 IT MI982569
(71) Applicant: Promefarm s.r.l., 20129 Milano (IT)
(72) Inventor: Ronchi, Celestino, 20127 Milano (IT); Ceschel, Giancarlo, 20121 Milano (IT)
(74) Representative: Marchi, Massimo, Dr.

(57) **Abstract**

A substantially anhydrous pharmaceutical composition comprising a nitric oxide (NO) donating compound, a mucoadhesive compound and an emulsifier capable of forming a microemulsion on addition of water; microemulsion thus obtained.

## Description

### FIELD OF THE INVENTION

The present invention relates to a substantially anhydrous pharmaceutical composition comprising a nitric oxide (NO) donating compound and to a pharmaceutical composition in the form of a microemulsion obtained from the said composition.

More particularly, the present invention relates to a substantially anhydrous pharmaceutical composition comprising a nitric oxide (NO) donating compound together with a mucoadhesive compound and an emulsifier.

Even more particularly, the present invention relates to a substantially anhydrous pharmaceutical composition for topical use, comprising a nitric oxide (NO) donating compound together with a mucoadhesive compound and an emulsifier.

The pharmaceutical composition of the present invention is particularly indicated in the treatment of complaints of the anorectal region, such as, for example, anal rhagades, haemorrhoids and transient proctalgia.

### BACKGROUND OF THE INVENTION

It is known that anal rhagades are ulceration of the mucosa or of the skin of the anal channel, which are accompanied by pain, which may be intense, in the anal region, especially during defecation, which is occasionally prevented, resulting in persistent constipation.

As for haemorrhoids, these are varicose dilations of the veins in the anal region, which result in pain and bleeding during defecation and, in many cases, pain and discomfort during daily activities.

Transient proctalgia, or spasm of the *levator ani* muscle, causes intense pain in the anorectal region.

These conditions are manifested in both sexes and are relatively common. The abovementioned complaints, the aetiopathogenesis of which has still not been fully established, have in common the impaired tonicity of the inner anal sphincter, which has an abnormally high pressure, often accompanied by spasms.

A satisfactory treatment for treating the abovementioned anorectal pathologies has not yet been found to date. For example, in the case of persistent anal rhagades, it is necessary to resort to surgical intervention, which is not without complications.

Nitric oxide (NO) donating compounds have been studied for a long time on account of the pharmacological properties of the nitric oxide (NO) released. The various pharmacological properties of NO which are known include its activity as a neurotransmitter. In particular, it plays an important role in mediating anorectal relaxation.

For this purpose, various studies have been carried out, including human studies, to develop a pharmaceutical composition based on nitric oxide (NO) donors which is useful in the treatment of the abovementioned anorectal pathologies.

For example, Guillemot et al. have shown that an appreciable reduction in the pressure of the anal sphincter is obtained by applying to the rectal mucosa an ointment containing nitroglycerine, which is a compound known as an NO donor. The said ointment was the one used percutaneously in the syndrome *angina pectoris* (sold under the trade name Lenitral®), suitably diluted ("Dis. Colon and Rectum", 36, 372, 1993).

These data were confirmed by Loder et al. who investigated, in patients with anal rhagades and haemorrhoids, the effect, on the tonus of the inner anal sphincter, of an external topical administration of another topical pharmaceutical composition containing nitroglycerine (sold under the trade name Percutol®), also suitably diluted. In this study also, a considerable decrease in the pressure in the anal sphincter was found ("British Journal of Surgery", 81 1386, 1994).

Lund and Scholefield carried out a controlled study in patients with anal rhagades, using the diluted composition Percutol®, and obtained encouraging results ("Lancet", 11, 349, 1997).

However, a certain percentage of patients, in all of the abovementioned studies, experienced strong headaches.

This phenomenon appears to be due to the systemic absorption of nitroglycerine.

In addition, with the type of products used by the abovementioned authors, a very rapid absorption of the nitroglycerine donor is obtained, thereby entailing the additional drawback of having to repeat the application of the preparation several times a day.

What is more, a known property of NO is its coronarodilatory effect. This effect has been known for some time, and is exploited in the treatment of *angina pectoris* by means of sublingual administration of nitroglycerine, which is immediately effective on the anginal pain.

In order to hinder the diffusion of nitroglycerine outside the area in which its action is required, it has been proposed to use sublingual nitroglycerine tablets containing bioadhesive substances (J.M. Schor et al., "Drug Dev. Ind. Pharm" 9 1359, 1983).

It is also known that, currently, mucoadhesive compounds are used in the pharmaceutical field for controlling the release of drugs. In general, mucoadhesive compounds are polymers with several hydrophilic functional groups capable of forming hydrogen bonds with the layer of mucin on mucous tissue ("Journal of Controlled Release", 12, 187-194, 1990).

The inventors of the present invention have investigated a composition containing an NO-donating compound, such as nitroglycerine, and a mucoadhesive compound, such as a polycarbiphil (see the composition of the comparative ointment in Example 9) to assess whether or not mucoadhesive compounds are capable of hindering the systemic absorption of nitroglycerine.

However, the results were unsatisfactory (see Comparative Test 1). The reason for this is that, despite the presence of the mucoadhesive compound, the nitroglycerine was absorbed systematically and its *in situ* persistence was short, as in the case of the formulation containing no mucoadhesive compound.

### AIMS OF THE INVENTION

The present invention proposes to provide a topical pharmaceutical composition capable of hindering the systemic absorption of a nitric oxide (NO) donating compound.

In particular, the present invention proposes to provide a topical composition which hinders the systemic absorption of a nitric oxide (NO) donating compound through mucous tissue, even more particularly through anorectal mucous tissue.

### DEFINITIONS

In the course of the present description and in the claims, the term "nitric oxide (NO) donating organic compound" means an organic compound capable of releasing nitric oxide.

Typical examples of nitric oxide (NO) donating organic compounds are organic esters of nitric acid, such as, for example, ethylene glycol dinitrate, isopropyl nitrate, glyceryl 1-mononitrate, glyceryl 1,2-dinitrate, glyceryl 1,3-dinitrate, nitroglycerine, butane-1,2,4-triol trinitrate, erythrityl tetranitrate, pentaerythrityl tetranitrate and isosorbide mononitrate, which can, in turn, comprise isosorbide 2-mononitrate, isosorbide 5-mononitrate and isosorbide dinitrate.

The term "mucoadhesive compound" means a polymer capable of adhering to the layer of mucin on mucous tissue. Many methods for measuring the force of mucoadhesion have been proposed to date. For the purposes of the present invention, the force of mucoadhesion is measured using an Instron Mod. 1114 tensile tester according to the method of Gurny et al. ("Biomaterials", 5, 336, 1984) and a compound is considered as being "mucoadhesive" when, at a concentration of 10% (w/w), the peeling force required is ≥ 1000 mN with an adhesion work of ≥ 0.5 mJ. In the case of the preferred mucoadhesive compounds, according to the present invention, the peeling force required is ≥ 1500 mN with an adhesion work of ≥ 0.7 mJ.

For an interesting review of mucoadhesion, see "Journal of Controlled Release", 2, 47-57, 1985; "International Journal of Pharmaceutics", 38, 65-70, 1987; "Journal of Controlled Release", 5, 129-141, 1987; "Journal of Controlled Release", 5, 143-149, 1987; "Indian J. Pharm. Sci.", 50(3), 145-152, 1988; "Drug Development and Industrial Pharmacy", 14(2&3), 283-318, 1988; "Journal of Controlled Release", 12, 187-194, 1990 and "Journel of Controlled Release", 31 207-212, 1994.

Typical examples of mucoadhesive compounds are polycarbophil, carboxymethylcellulose, polyacrylic acid, hyaluronic acid, gum tragacanth, maleic anhydride-co-polymethyl vinyl ether, polyethylene oxide, methylcellulose, sodium alginate, hydroxypropyl-methylcellulose, karaya gum, methylethylcellulose, soluble starch, gelatin, pectin, polyvinylpyrrolidone, polyethylene glycol, polyvinyl alcohol, polyhydroxyethyl methacrylate, hydroxypropylcellulose, *Cyanopsis tetragonaloba* gum and gum arabic.

The term "emulsifier" is used to indicate a substance capable of spontaneously forming a microemulsion of the "oil-in-water" type by addition of at least 0.01% (w/w) of water, in particular in the presence of at least 0.01% (w/w) of water.

Typical examples of emulsifiers are sucrose mono- and distearate, sucrose monopalmitate, polyethylene glycol-300 stearate, ethylene glycol and polyoxyethylene glycol palmitostearate, polyoxyethylene glycol palmitostearate, polyethylene glycol palmitostearate combined with a polyoxyethylenated fatty alcohol, glyceryl and polyethylene glycol palmitostearate combined with an ethoxylated fatty alcohol, propylene glycol palmitostearate combined with an amine soap, rectified diethylene glycol monoethyl ether, saturated C₈₋₁₀ polyglycosilated glycerides, saturated C₈₋₁₀ polyglycosilated glycerides, caprylic and capric acid triglycerides, polyglycosilated isostearic glycerides, unsaturated polyglycosilated glycerides from apricot kernel oil, unsaturated polyglycosilated glycerides from olive oil, unsaturated polyglycosilated glycerides from corn oil, saturated polyglycosilated glycerides from palm oil and from palm kernel oil, saturated polyglycosilated glycerides from hydrogenated castor oil, unsaturated polyglycosilated glycerides from corn oil and saturated C₈₋₁₀ polyglycosilated glycerides.

The term "microemulsion" is used to indicate a system capable of enveloping water spontaneously, i.e. at room temperature and in the absence of shear forces, forming an emulsion of the oil-in-water type.

The expression "substantially anhydrous pharmaceutical composition" is used to indicate a composition whose weight remains constant after heating for 1 hour in an oven with hot air at 40°C under vacuum. The capacity of this composition to form a microemulsion is assessed by adding 3 parts by weight of FU purified water per one part by weight of composition, at about 25°C and with gentle stirring for not more than 2 minutes. This should thus give a homogeneous emulsion of the oil-in-water type in which the average size of the particles of oily phase dispersed in water is not greater than 10-20 µm (microscope).

### SUMMARY OF THE INVENTION

The present invention is based on the observation that the co-presence of a mucoadhesive compound and of an emulsifier produces, surprisingly, a synergistic effect with regard to hindering the systemic absorption of a nitric oxide (NO) donating compound. In particular the absorption of an NO-donating compound through mucous tissue, even more particularly through anorectal mucous tissue.

It is, therefore, a first object of the present invention to provide a substantially anhydrous pharmaceutical composition comprising a nitric oxide (NO) donating compound, characterized in that it further comprises a mucoadhesive compound and an emulsifier and is capable of forming a microemulsion on addition of water.

Preferably:
- the nitric oxide (NO) donating compound is nitroglycerine;
- the mucoadhesive compound is a polycarbophil;
- the emulsifier is chosen from the group comprising unsaturated polyglycosilated glycerides from apricot kernel oil, saturated polyglycosilated glycerides from hydrogenated castor oil and diethylene glycol monoethyl ether.

Preferably, the composition of the present invention comprises between 0.01 and 6%, even more typically between 0.1 and 2%, of NO-donating compound.

Typically, the amount of mucoadhesive compound is between 0.1 and 30%, even more typically between 1 and 10%.

The amount of emulsifier is typically between 1 and 60%, even more typically between 5 and 45%.

Preferably, the composition of the present invention is suitable for topical use, even more preferably for internal topical use.

Examples of suitable dosage forms are medicated plasters, creams, gels, ointments, suppositories, enemas and anal foams.

Preferably, the composition of the present invention is in the form of an ointment or a suppository and, besides the abovementioned ingredients, also comprises at least one excipient or one vehicle of conventional type.

The said dosage forms can also contain other conventional ingredients such as, for example, preserving agents, diluents, antioxidants, chelating agents, propellants, technological co-adjuvants, colourants and the like.

The dosage forms of the pharmaceutical composition of the present invention can be prepared according to techniques which are well known to pharmaceutical chemists, comprising conventional operations such as mixing, melting, enveloping, homogenization and the like.

Typical examples of pathological states which can gain benefit from the treatment with a pharmaceutical composition according to the present invention are haemorrhoids, anal ulcers and rhagades.

The present invention is described further with the aid of the Tests and Examples which follow, which are given purely for illustrative purposes and should not be considered as limiting the invention.

### EXAMPLE 1

### Ointment A

| Components: | grams |
|---|---|
| Nitroglycerine | 2 |
| Miglyol 812 (medium-chain unsaturated triglycerides; excipient) | 38 |
| Labrafil M 1944 CS (unsaturated polyglycosilated glycerides from apricot kernel oil; emulsifier) | 45 |
| Ceresin (excipient) | 10 |
| Noveon AA1 (polycarbophil; mucoadhesive compound) | 5 |

The nitroglycerine, the Labrafil M 1944 CS (from the company Gatte Fosse, Saint Priest, France) and the Miglyol 812 (from the company Hüls AG, Marl, Germany) were placed in a spherical turbo-emulsifier from the company Comer - Erba (Cernusco sul Naviglio, Milan, Italy) and were mixed for 20 minutes at 40°C. The Ceresin (from the company SBP Ltd., London, England), premelted at 80°C, was then added to this mixture.

The mixture was again brought to 40°C, followed by addition of the Noveon AA1 (from the company B.F. Goodrich Chemical, Milan, Italy) and the homogenization phase was started at the maximum speed of the turbo-emulsifier for about 10 minutes.

The homogenisate thus obtained was cooled under vacuum to 25°C and finally divided into 30 g aluminium tubes lined with epoxy resin.

### EXAMPLE 2

### Ointment B

| Components: | grams |
|---|---|
| Nitroglycerine | 2 |
| Miglyol 812 | 38 |
| Labrafil M 1944 CS | 5 |
| Ceresin | 10 |
| Noveon AA1 | 5 |
| Transcutol (rectified diethylene glycol monoethyl ether; emulsifier) | 20 |
| PEG-400 (polyethylene glycol; excipient) | 20 |

The process was performed as described in Example 1 above, except that the composition also contained Transcutol (from the company Gatte Fosse, Saint Priest, France) and PEG-400 (from the company BASF, Cheshire, England).

### EXAMPLE 3

### Ointment C

| Components: | grams |
|---|---|
| Nitroglycerine | 2 |
| Miglyol 812 | 38 |
| PEG-1500 (polyethylene glycol; excipient) | 30 |
| Ceresin | 5 |
| Noveon AA1 | 5 |
| Transcutol | 20 |

The process was performed as in Example 1 above, except that the amount of Ceresin was reduced and PEG-1500 (from the company BASF, Cheshire, England) was added instead of the Labrafil M 1944 CS.

### EXAMPLE 4

### Ointment D

| Components: | grams |
|---|---|
| Nitroglycerine | 0.2 |
| Miglyol 812 | 30 |
| Ceresin | 10 |
| Noveon AA1 | 3 |
| Labrafil M 1944 CS | qs 100 |

The process was performed as in Example 1 above, except that the amount of nitroglycerine, Miglyol 812 and Noveon AA1 was reduced and the amount of Labrafil M 1944 CS was increased.

### EXAMPLE 5

### Ointment E

| Components: | grams |
|---|---|
| Nitroglycerine | 0.1 |
| Labrafil M 1944 CS | 5 |
| PEG-400 | 10 |
| Ceresin | 10 |
| Noveon AA1 | 5 |
| Transcutol | 15 |
| White petroleum jelly | qs 100 |

The process was performed as in Example 1 above, except that the amount of nitroglycerine, of Labrafil M 1944 CS and of Ceresin was reduced and PEG-400 and white petroleum jelly were added instead of the Miglyol 812.

### EXAMPLE 6

### Ointment F

| Components: | grams |
|---|---|
| Nitroglycerine | 0.6 |
| PEG-1500 | 30 |
| Ceresin | 5 |
| Noveon AA1 | 5 |
| Transcutol | 20 |
| Miglyol 812 | qs 100 |

The process was performed as in Example 1 above, except that the amount of nitroglycerine and of Ceresin was reduced, the amount of Miglyol 812 was increased and PEG-1500 and Transcutol were added instead of the Labrafil M 1944 CS.

### EXAMPLE 7

### Suppository

| Components: | milligrams |
|---|---|
| Nitroglycerine | 10 |
| Lactose | 200 |
| Noveon AA1 | 50 |
| Labrafil WL 1958 CS (saturated polyglycosilated glycerides from hydrogenated castor oil; emulsifier) | 200 |
| Suppocire BS2X (semisynthetic glycerides; excipient) | qs 3 g |

The Suppocire BS2X (from the company Gatte Fosse, Saint Priest, France) was melted at 50°C and the Labrafil WL 1958 CS (from the company Gatte Fosse, Saint Priest, France) and the nitroglycerine absorbed on lactose were added.

The mixture was then homogenized at 45°C using a Rup Alfred Brogli & Co. model Alleinvertrieb homogenizer (from the company Tecnisches Bureau, Basle, Switzerland). The homogenisate thus obtained was collected and placed in the hopper of a suppository distributor from the company Sarong (Bologna, Italy) and distributed therein at 38°C to form suppositories of 3 g each.

### EXAMPLE 8

### Comparative ointment I

| Components: | grams |
|---|---|
| Nitroglycerine | 2 |
| Miglyol 812 | 38 |
| Paraffin wax | 4 |
| Amerchol CAB (petrolatum and lanolin alcohol; excipient) | 10 |
| White petroleum jelly | 46 |

The white petroleum jelly, the Amerchol CAB (from the company Amerchol Europe, Vilvoorde, Belgium) and the paraffin wax were placed in a spherical turbo-emulsifier thermostatically adjusted to 70°C and the mixture was then brought to the melting point. This molten mixture was then cooled to 40°C and the nitroglycerine diluted in Miglyol 812 was added.

The mixture thus obtained was cooled at a rate of 1°C/10 minutes and at 80 mm Hg.

Finally, the atmosphere was replaced with fresh and the ointment thus obtained was then divided into 30 g aluminium tubes lined with epoxy resin.

### EXAMPLE 9

### Comparative ointment II

| Components: | grams |
|---|---|
| Nitroglycerine | 2 |
| Miglyol 812 | 38 |
| Noveon AA1 | 4 |
| Paraffin wax | 4 |
| Amerchol CAB | 10 |
| White petroleum jelly | 42 |

The process was performed as in Example 8 above, except that, before the cooling phase, the Noveon AA1 was added and dispersed.

### EXAMPLE 10

### Comparative ointment III

| Components: | grams |
|---|---|
| Nitroglycerine | 2 |
| Miglyol 812 | 38 |
| Transcutol | 25 |
| PEG-1500 | 30 |
| Ceresin | 5 |

The process was performed as in Example 8 above, except that the Transcutol, the PEG-1500 and the Ceresin were also added to the turbo-emulsifier.

### TEST 1

### ex-vivo Test of transmembrane permeability

The transmembrane permeability of the compositions of the present invention was assessed by means of the technique of Franz diffusion cells, which is considered as being predictive of the absorption through human mucosa (Th. Franz & al., "The Journal of Investigative Dermatology", 64, 190-195, 1975; Th. Franz & al., "Current problem of dermatology" 7 58-68, 1978; Feldmann & Mainbach, "The Journal of Investigative Dermatology, 54, 399-404, 1970).

A Franz cell from the company Crow Glass Company Inc. (New Jersey, USA) was used, composed of a donor compartment (9 mm in diameter, corresponding to a diffusion area of 64 mm²) and a receiver compartment (with a capacity of 4.8 ml), in which the receiver compartment was surrounded by a jacket in which water thermostatically maintained at 37 ± 1 °C flowed.

A sample of mucous tissue was prepared, using oral mucosa from pigs, which are similar to human mucosa in terms of structure and permeability (R. Bronaugh & R. Stewart, "Toxicology and Applied Pharmacology", 62, 481-488, 1982). A square-shaped sample of mucous tissue (with a side length of 2 cm and 0.9 - 1.1 mm in thickness) was thus prepared and was then placed between the receiver compartment and the donor compartment and, finally, the assembly was hermetically closed with spring pliers.

The sample of tissue was covered with 1 g of a composition of the present invention (ointment A, B and C) comprising 20 mg of nitroglycerine.

Phosphate buffer (4.8 ml, pH 7.4) thermostatically maintained at 37 ± 1°C was introduced into the receiver compartment.

Samples of solution were taken from the receiver compartment at predetermined time intervals. After 0 and 30 minutes, 1 ml of solution was taken, and 4 ml were taken in the subsequent samplings (after 1, 2, 4, 6 and 8 hours). The amount of nitroglycerine in the solution samples taken was determined by HPLC.

The results obtained are given in the following table.

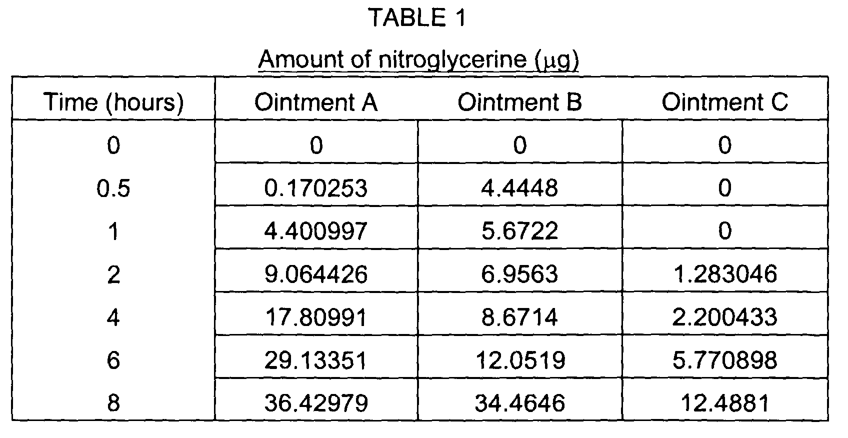

### COMPARATIVE TEST 1

The capacity for permeation, across a mucous membrane, of nitroglycerine contained in the Comparative Ointments I, II and III was determined as described in Test 1 above.

Results thus obtained are given in Table 2.

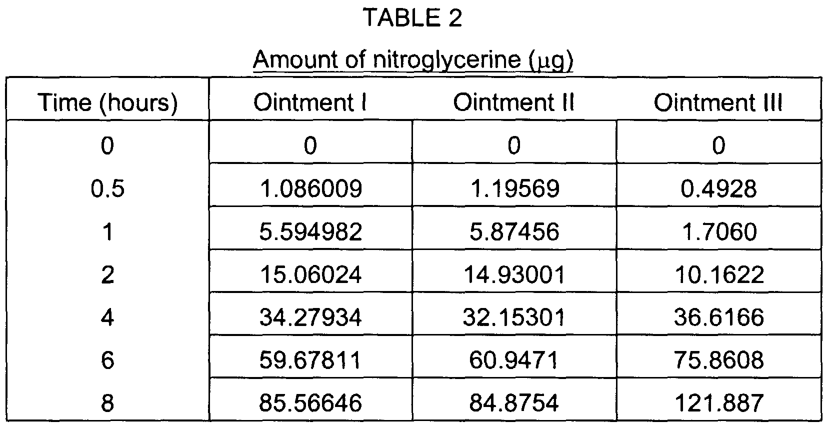

The data reported in Tables 1 and 2 show that the compositions of the present invention are capable of considerably hindering the absorption of nitroglycerine, either with respect to the conventional ointment (Comparative Ointment I) or with respect to ointments which contain only the mucoadhesive compound (Comparative Ointment II) or only the emulsifier (Comparative Ointment III).

The reduction in the systemic absorption of nitroglycerine by the composition of the present invention was confirmed in humans by means of preliminary tolerability tests.

## Claims

1. Substantially anhydrous pharmaceutical composition comprising a nitric oxide (NO) donating compound, characterized in that it further comprises a mucoadhesive compound and an emulsifier and is capable of forming a microemulsion on addition of water.

2. Composition according to Claim 1, characterized in that the nitric oxide (NO) donating compound is nitroglycerine.

3. Composition according to Claim 1 or 2, characterized in that the mucoadhesive compound is a polycarbophil.

4. Composition according to any one of the preceding Claims 1 to 3, characterized in that the emulsifier is chosen from the group comprising unsaturated polyglycosilated glycerides from apricot kernel oil, saturated polyglycosilated glycerides from hydrogenated castor oil and rectified diethylene glycol monoethyl ether, and mixtures thereof.

5. Composition according to any one of the preceding Claims 1 to 4, characterized in that it comprises between 0.01 and 6% of NO-donating compound.

6. Composition according to Claim 5, characterized in that it comprises between 0.1 and 2% of NO-donating compound.

7. Composition according to any one of the preceding Claims 1 to 6, characterized in that it comprises between 0.1 and 30% of mucoadhesive compound.

8. Composition according to Claim 7, characterized in that it comprises between 1 and 10% of mucoadhesive compound.

9. Composition according to any one of the preceding Claims 1 to 8, characterized in that it comprises between 1 and 60% of emulsifier.

10. Composition according to Claim 9, characterized in that it comprises between 5 and 45% of emulsifier.

11. Composition according to any one of the preceding Claims 1 to 10, characterized in that it is for topical use.

12. Microemulsion of the oil-in-water type, characterized in that it is obtained by addition of water to a composition according to any one of the preceding Claims 1 to 11.
